# EUROPEAN PATENT APPLICATION

(11) **EP 4 831 700 A1**
(43) Date of publication of application: **28.10.2026**
(21) Application number: 24906242.3
(22) Date of filing: 12.12.2024
(51) Int. Cl.: G02B 5/18, C07C 69/612, C07C 15/46, C08F 220/10, C08F 212/08

(54) **VOLUME HOLOGRAPHIC GRATING RAW MATERIAL SYSTEM, AND VOLUME HOLOGRAPHIC GRATING AND PREPARATION METHOD THEREFOR**

(30) Priority: 21.12.2023 CN 202311776170
(71) Applicant: Meta-Bounds Inc., Zhuhai, Guangdong 519000 (CN)
(72) Inventor: GUAN, Jian, Zhuhai, Guangdong 519000 (CN); LAN, Fuyang, Zhuhai, Guangdong 519000 (CN); WANG, Zhaomin, Zhuhai, Guangdong 519000 (CN)
(74) Representative: Agca Kizil, Tugce
(86) International application number: PCT/CN2024/138935
(87) International publication number: WO 2025/130759

(57) **Abstract**

The present disclosure relates to the field of display technologies, and in particular to a raw material system for a volume holographic grating, a volume holographic grating and a preparation method for a volume holographic grating. The raw material system for the volume holographic grating includes a photopolymerizable monomer, a liquid crystal, an organic additive and a photoinitiator. The organic additive is a compound of Formula . A is a group having affinity for photopolymerizable monomers, and B is a group having affinity for liquid crystals. Based on the raw material system of the present disclosure, a novel organic additive is introduced. The organic additive facilitates favorable phase separation of a PDLC system.

## Description

This application is a National Stage of International Application No. PCT/CN2024/138935, filed on December 12, 2024, which claims priority to Chinese patent application No. 202311776170.4, filed with China National Intellectual Property Administration on December 21, 2023 and entitled "Raw Material System for a Volume Holographic Grating, Volume Holographic Grating and Preparation Method for a Volume Holographic Grating", the entire contents of which are incorporated herein by reference.

### Technical Field

The present disclosure relates to the field of display technologies, and in particular to a raw material system for a volume holographic grating, a volume holographic grating and a preparation method for a volume holographic grating.

### Background Art

As coupling optical elements of holographic optical waveguides, Volume Holographic Gratings (VHG) can be applied to display technologies such as Virtual Reality (VR), Augmented Reality (AR), Mixed Reality and Head-Up Display. The performance of a VHG is measured by refractive index modulation (Δn) and diffraction efficiency (DE). The higher the two values, the better the light modulation performance of VHG. Organic VHG material systems are generally divided into two categories: photo-induced second-order polymers and Holographic Polymer Dispersed Liquid Crystal (HPDLC). Since the Δn of HPDLC material system can exceed 0.1 or higher, far exceeding that of photo-induced second-order polymers (Δn is 0.04), it is currently the optimal material system for a volume holographic grating. However, performance improvement based on this material system has stagnated for years. There is an urgent need to develop methods to improve Δn to meet the requirements of display applications in AR/VR fields.

In the HPDLC material system, the main components are photopolymerizable monomers, photoinitiators and liquid crystal components. These components can be uniformly mixed and coated to form a thin film. When two beams of interference light irradiate the thin film, photopolymerization occurs in a bright region while no reaction takes place in a dark region. At this time, photopolymerizable monomers and liquid crystal components gradually change from a uniform mixed state, with photopolymerizable monomers diffusing toward the bright region and liquid crystal components diffusing toward the dark region. Finally, polymer-liquid crystal-polymer regions are formed in the order of bright-dark-bright. The greater the difference between the refractive index of the polymer and the refractive index of the liquid crystal, the greater the refractive index modulation of the volume holographic grating. Two conventional approaches are adopted to increase Δn, namely reducing the refractive index of polymers or increasing the refractive index of liquid crystals.

Scheme 1: Reduce the refractive index of polymers. Shi Mengquan et al. designed and synthesized low-refractive-index olefin monomers, such as acrylic acid segments and styrene segments. Such low-refractive-index olefin monomers were applied to photopolymer material systems to prepare holographic storage materials (see patent document No. CN02149193.3 for details).

Scheme 2: Increase the refractive index of liquid crystals. Digilens Inc. blended high-refractive-index and low-refractive-index liquid crystals to increase the average refractive index of liquid crystals. The refractive index of a high-refractive-index liquid crystal can reach 1.7, and volume holographic gratings with Δn greater than 0.1 were accordingly fabricated (see patent document No. US2020/0271973A1 for details).

However, the above schemes involve simply changing the refractive index of polymers or the refractive index of liquid crystals. Simply changing the refractive index of the polymers or the refractive index of the liquid crystals will cause significant changes in the surface energy, viscosity, film-forming properties and other properties of corresponding components, further altering the compatibility between polymers and liquid crystals. In other words, a change in a single component will lead to re-proportioning of all components in the entire system, which severely reduces the optimization efficiency of the refractive index modulation.

Therefore, the related art still needs further improvement and development.

### Summary of the Invention

In view of the deficiencies of the related art, the present disclosure aims to provide a raw material system for a volume holographic grating, a volume holographic grating and a preparation method for a volume holographic grating, so as to solve the problem that the existing methods for improving the refractive index modulation of volume holographic gratings by changing a single component still have insufficient improvement effect.

The technical solutions of the present disclosure are as follows.

In a first aspect, the present disclosure provides a raw material system for a volume holographic grating, including a photopolymerizable monomer, a liquid crystal, an organic additive and a photoinitiator; wherein the organic additive is a compound of Formula A-B, wherein A is a group having affinity for photopolymerizable monomers, and B is a group having affinity for liquid crystals.

Optionally, the group having affinity for photopolymerizable monomers is an ester group, and the group having affinity for liquid crystals is selected from the group consisting of phenyl group, fluoro-substituted phenyl group, cyano-substituted phenyl group, trifluoromethyl-substituted phenyl group, methyl-substituted phenyl group, thienyl group, pyrrolyl group, cyano-substituted alkyl group and halogen-substituted alkyl group.

Further optionally, the organic additive is one or more compounds selected from the group consisting of Formulas:

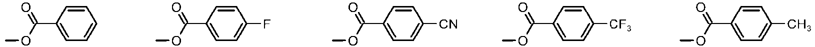

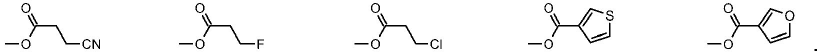

Optionally, the group having affinity for photopolymerizable monomers is selected from the group consisting of alkene-containing group, carbonyl-containing group and alkyl ether group, and the group having affinity for liquid crystals is phenyl group.

Further optionally, the alkene-containing group is an allyl group, and the carbonyl-containing group is selected from the group consisting of ethyl ketone group, acetaldehyde group and acetamido group.

Still further optionally, the organic additive is one or more compounds selected from the group consisting of Formulas:

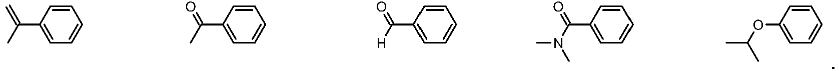

Optionally, based on the total weight of the raw material system for the volume holographic grating, the weight percentage of the photopolymerizable monomer is 30%-50%, the weight percentage of the liquid crystal is 30%-50%, the weight percentage of the organic additive is 1%-10%, and the weight percentage of the photoinitiator is 1%.

Optionally, the photopolymerizable monomer is at least one selected from the group consisting of acrylates and acrylate derivatives;
the liquid crystal is a nematic liquid crystal; and
the photoinitiator is a mixture of dibromofluorescein and phenylglycine, or a mixture of Acid Red 94 and phenylglycine.

In a second aspect, the present disclosure provides a volume holographic grating prepared by subjecting the aforementioned raw material system for the volume holographic grating to coherent light exposure.

In a third aspect, the present disclosure provides a preparation method for a volume holographic grating, comprising: subjecting the aforementioned raw material system for the volume holographic grating to coherent light exposure to obtain the volume holographic grating.

Beneficial effects: Based on the raw material system composed of the photopolymerizable monomer, the liquid crystal and the photoinitiator, a novel organic additive is introduced in the present disclosure. The organic additive facilitates favorable phase separation of a PDLC (Polymer Dispersed Liquid Crystal) system, increases the refractive index difference between the bright region and the dark region, thus improving the refractive index modulation of the volume holographic grating to meet the requirements of display applications in AR/VR fields.

### Brief Description of the Drawings

FIG. 1 is a schematic diagram showing the formation of a periodic grating after a raw material system containing a photoinitiator, liquid crystal and a photopolymerizable monomer is coated into a film and then exposed to coherent light.
FIG. 2 is a schematic diagram showing the principle of an organic additive promoting phase separation of a PDLC film.
FIG. 3 shows the test results of the refractive index modulation of volume holographic gratings for Embodiments 1-3 and Comparative Example 1.

### Detailed Description of Embodiments

The present disclosure provides a raw material system for a volume holographic grating, a volume holographic grating and a preparation method for a volume holographic grating. To make the objectives, technical solutions and effects of the present disclosure clearer and more definite, the present disclosure is described in further detail below. It should be understood that the specific embodiments described herein are used to explain the present disclosure, and are not intended to limit the present disclosure.

Given the great difficulty in optimizing the refractive index modulation of existing volume holographic gratings, which involves complex molecular design and delivers marginal performance improvement, the present disclosure uses a novel additive that can significantly promote phase separation between polymer and liquid crystal components and optimize the refractive index modulation of the polymer and liquid crystal components.

Specifically, an embodiment of the present disclosure provides a raw material system for a volume holographic grating. The raw material system for the volume holographic grating includes a photopolymerizable monomer, a liquid crystal, an organic additive and a photoinitiator. The organic additive is a compound of Formula A-B. A is a group having affinity for photopolymerizable monomers and B is a group having affinity for liquid crystals.

It should be noted that the group having affinity for photopolymerizable monomers refers to a group having good affinity for photopolymerizable monomers, or a group capable of attracting photopolymerizable monomers. The group having affinity for liquid crystals refers to a group having good affinity for liquid crystals, or a group capable of attracting liquid crystals.

Based on the raw material system composed of the photopolymerizable monomer, the liquid crystal and the photoinitiator, and an organic additive is introduced in an embodiment of the present disclosure. The organic additive can promote phase separation of a PDLC (Polymer Dispersed Liquid Crystal) film during exposure, increase the refractive index difference between the bright region and the dark region, thus improving the refractive index modulation of the volume holographic grating to meet the requirements of display applications in AR/VR fields.

The organic additive introduced in the embodiments of the present disclosure has the characteristics of simple structure, expandable structure, and good light/thermal stability. Adding the organic additive into PDLC components can significantly improve the Δn value. Compared with conventional methods, the embodiments of the present disclosure do not require complex molecular design or lengthy multi-component optimization, have better universality, and can further improve the optical performance of the volume holographic grating.

The phase separation process of the PDLC system in the embodiments of the present disclosure and the fundamental mechanism of phase separation promotion by the introduced organic additive are elaborated as follows.

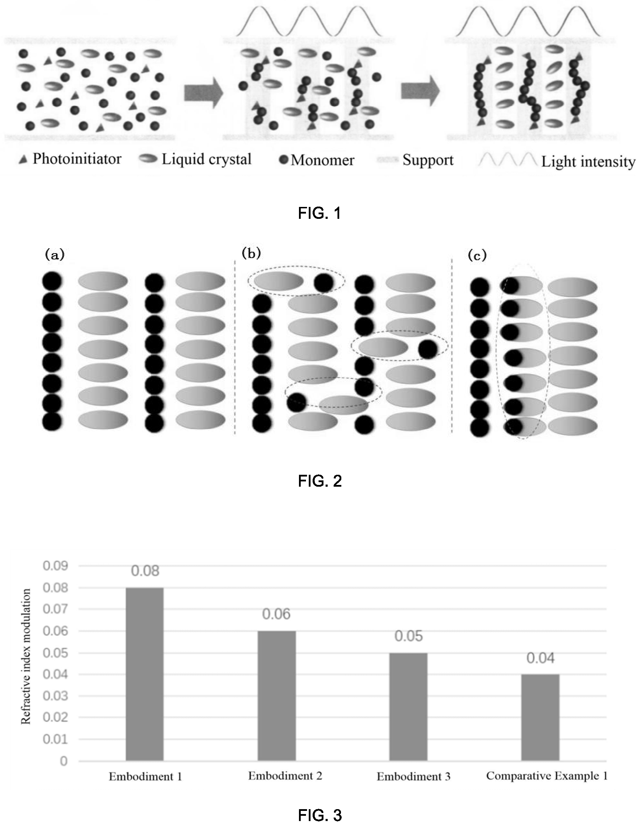

The phase separation process of the PDLC system can be briefly divided into the following stages: ① As shown in FIG. 1, the raw material system is first coated between supports. At this time, the photoinitiator, the liquid crystal and the photopolymerizable monomer are uniformly mixed and randomly distributed in the film. ② When coherent light is used for exposure, different physicochemical processes occur in alternating bright and dark fringes. In the coherent bright region, the photoinitiator absorbs photons and generates active centers, triggering polymerization of photopolymerizable monomers to form polymer (polymer network), while squeezing the liquid crystal into the coherent dark region. No polymerization reaction occurs in the coherent dark region. Periodic gratings with polymer-rich and liquid crystal-rich phases are finally formed in the PDLC film.

The basic principle of organic additives promoting phase separation of the PDLC film: The ideal microstructure of the volume holographic grating is shown in FIG. 2(a), which consists of periodically and orderly distributed polymer phases and liquid crystal phases. However, in actual production, it is difficult to achieve the ideal effect. FIG. 2(b) shows a common defective structure where the distribution of polymers and liquid crystals deviates from the ideal state, with polymer mixed into the liquid crystal phase or liquid crystal mixed into the polymer phase, as shown in a dashed circle. This defect is often referred to as insufficient phase separation, which leads to a reduction in refractive index modulation. To address this defect, an organic additive (as shown in a dashed circle of FIG. 2(c)) is introduced in an embodiment of the present disclosure. The organic additive includes characteristic groups with affinity for photopolymerizable monomers and other characteristic groups with affinity for liquid crystals. During exposure, the organic additive acts as an intermediate component between the liquid crystal phase and the polymer phase to alleviate insufficient diffusion of liquid crystals and polymers. This principle is realized based on the like-dissolves-like theory, which states that liquid crystals are more compatible with liquid crystals and photopolymerizable monomers are more compatible with photopolymerizable monomers. The introduction of the organic additive reduces the probability of disordered distribution of liquid crystals and polymers, thus improving the refractive index modulation.

In one embodiment, the group having affinity for photopolymerizable monomers is an ester group. The group having affinity for liquid crystals is selected from the group consisting of phenyl group, fluoro-substituted phenyl group, cyano-substituted phenyl group, trifluoromethyl-substituted phenyl group, methyl-substituted phenyl group, thienyl group, pyrrolyl group, cyano-substituted alkyl group and halogen-substituted alkyl group.

As an example, the organic additive is one or more compounds selected from the group consisting of Formulas:

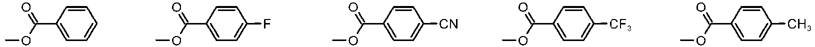

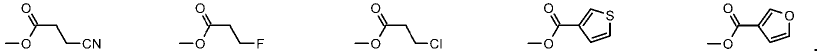

In one embodiment, the group having affinity for photopolymerizable monomers may be selected from the group consisting of alkene-containing group, carbonyl-containing group and alkyl ether group. The group having affinity for liquid crystals may be a phenyl group. The alkene-containing group may be an allyl group. The carbonyl-containing group may be selected from the group consisting of ethyl ketone group, acetaldehyde group and acetamido group.

The above groups have strong polarity. Carbonyl groups and oxygen atoms exhibit good compatibility with common acrylate monomers. During the phase separation process, they act as effective intermediate segments between photopolymerizable monomers and liquid crystals, increasing the degree of phase separation.

As an example, the organic additive is one or more compounds selected from the group consisting of Formulas:

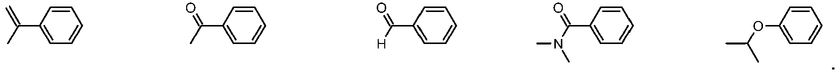

In one embodiment, based on the total weight of the raw material system for the volume holographic grating, the weight percentage of the photopolymerizable monomer is 30%-50%, the weight percentage of the liquid crystal is 30%-50%, the weight percentage of the organic additive is 1%-10%, and the weight percentage of the photoinitiator is 1%. By controlling the proportion of each component in the raw material system for the volume holographic grating, the refractive index modulation of the volume holographic grating can be effectively improved.

In one embodiment, the photopolymerizable monomer may be at least one conventional photopolymerizable monomer in the art, including but not limited to acrylates and acrylate derivatives.The acrylate derivatives include at least one of 2-hydroxy-3-acrylic acid propyl ester, methyl acrylate, methyl methacrylate, deuterated methyl methacrylate, fluoro-substituted methyl methacrylate, ethyl acrylate, ethyl methacrylate, ethyl ethyl acrylate, propyl ethyl acrylate, etc.

In one embodiment, the liquid crystal may be a common nematic liquid crystal, such as 4-cyano-4'-pentylbiphenyl, 5-octylphenyl-2-methoxyphenyl-4-pyridone, liquid crystal mixture E7 ( from Merck), etc.

In one embodiment, the photoinitiator may be a conventional photosensitive system sensitive to light in the red/blue bands. After photosensitization, the photoinitiator releases free radicals, which initiate the polymerization reaction of photopolymerizable monomers. For example, the photoinitiator may be dibromofluorescein/phenylglycine (a mixture of dibromofluorescein and phenylglycine) or Acid Red 94/phenylglycine (a mixture of Acid Red 94 and phenylglycine), etc.

An embodiment of the present disclosure provides a volume holographic grating prepared by subjecting the aforementioned raw material system for the volume holographic grating to coherent light exposure.

An embodiment of the present disclosure provides a preparation method for a volume holographic grating, which includes: subjecting the aforementioned raw material system for the volume holographic grating to coherent light exposure to obtain the volume holographic grating.

The raw material system for the volume holographic grating in an embodiment of the present disclosure is based on the raw material system composed of the photopolymerizable monomer, the liquid crystal and the photoinitiator, and introduces an organic additive. The organic additive can promote phase separation of a PDLC (Polymer Dispersed Liquid Crystal) film during exposure, increase the refractive index difference between the bright region and the dark region, thus improving the refractive index modulation of the volume holographic grating to meet the requirements of display applications in AR/VR fields.

The present disclosure will be described in detail below through specific embodiments.

### Embodiment 1

In this embodiment, the total weight of the raw material system for the volume holographic grating is 10 g. Methyl benzoate with nuclear magnetic resonance (NMR) purity of 99% is selected as an organic additive, with an addition amount of 5% (% is a mass percentage, the same in subsequent embodiments). 5-octylphenyl-2-methoxyphenyl-4-pyridone is used as a liquid crystal component, with an addition amount of 45%. 2-hydroxy-3-acrylic acid propyl ester is used as a photopolymerizable monomer, with an addition amount of 45%. Dibromofluorescein/phenylglycine is used as a photoinitiator, with an addition amount of 1%. Hexanoic acid is added as a plasticizer to maintain coating uniformity, with an addition amount of 4%.

Place the raw material system for the volume holographic grating in a round-bottom flask and stir with a magnetic stirrer for 10 minutes to ensure uniform mixing. Subsequently, the uniformly mixed raw material system for the volume holographic grating is filled into a liquid crystal cell by capillary action. The liquid crystal cell includes at least one glass substrate. After edge-sealing the liquid crystal cell, a standard sample is obtained. The standard sample is then exposed with a standard holographic exposure device. After exposing to red light for 30 seconds, a volume holographic grating is prepared. The refractive index modulation of the prepared volume holographic grating is measured via an optical platform.

### Embodiment 2

In this embodiment, the total weight of the raw material system for the volume holographic grating is 10 g. Methyl 1-cyanopropanoate with nuclear magnetic resonance purity of 99% is selected as an organic additive, with an addition amount of 5%. 5-octylphenyl-2-methoxyphenyl-4-pyridone is used as a liquid crystal component, with an addition amount of 45%. 2-hydroxy-3-acrylic acid propyl ester is used as a photopolymerizable monomer, with an addition amount of 45%. Dibromofluorescein/phenylglycine is used as a photoinitiator, with an addition amount of 1%. Hexanoic acid is added as a plasticizer to maintain coating uniformity, with an addition amount of 4%.

Place the raw material system for the volume holographic grating in a round-bottom flask and stir with a magnetic stirrer for 10 minutes to ensure uniform mixing. Subsequently, the uniformly mixed raw material system for the volume holographic grating is filled into a liquid crystal cell by capillary action. The liquid crystal cell includes at least one glass substrate. After edge-sealing the liquid crystal cell, a standard sample is obtained. The standard sample is then exposed with a standard holographic exposure device. After exposing to red light for 30 seconds, a volume holographic grating is prepared. The refractive index modulation of the prepared volume holographic grating is measured via an optical platform.

### Embodiment 3

In this embodiment, the total weight of the raw material system for the volume holographic grating is 10 g. α-methylstyrene with nuclear magnetic resonance purity of 99% is selected as an organic additive, with an addition amount of 5%. 5-octylphenyl-2-methoxyphenyl-4-pyridone is used as a liquid crystal component, with an addition amount of 45%. 2-hydroxy-3-acrylic acid propyl ester is used as a photopolymerizable monomer, with an addition amount of 45%. Dibromofluorescein/phenylglycine is used as a photoinitiator, with an addition amount of 1%. Hexanoic acid is added as a plasticizer to maintain coating uniformity, with an addition amount of 4%.

Place the raw material system for the volume holographic grating in a round-bottom flask and stir with a magnetic stirrer for 10 minutes to ensure uniform mixing. Subsequently, the uniformly mixed raw material system for the volume holographic grating is filled into a liquid crystal cell by capillary action. The liquid crystal cell includes at least one glass substrate. After edge-sealing the liquid crystal cell, a standard sample is obtained. The standard sample is then exposed with a standard holographic exposure device. After exposing to red light for 30 seconds, a volume holographic grating is prepared. The refractive index modulation of the prepared volume holographic grating is measured via an optical platform.

### Comparative Example 1

The total weight of the raw material system for the volume holographic grating in the comparative example is 10 g. 5-octylphenyl-2-methoxyphenyl-4-pyridone is used as a liquid crystal component, with an addition amount of 45%. 2-hydroxy-3-acrylic acid propyl ester is used as a photopolymerizable monomer, with an addition amount of 45%. Dibromofluorescein/phenylglycine is used as a photoinitiator, with an addition amount of 1%. Hexanoic acid is added as a plasticizer to maintain coating uniformity, with an addition amount of 9%.

Place the raw material system for the volume holographic grating in a round-bottom flask and stir with a magnetic stirrer for 10 minutes to ensure uniform mixing. Subsequently, the uniformly mixed raw material system for the volume holographic grating is filled into a liquid crystal cell by capillary action. The liquid crystal cell includes at least one glass substrate. After edge-sealing the liquid crystal cell, a standard sample is obtained. The standard sample is then exposed with a standard holographic exposure device. After exposing to red light for 30 seconds, a volume holographic grating is prepared. The refractive index modulation of the prepared volume holographic grating is measured via an optical platform.

### Result Analysis

The test results are shown in FIG. 3. FIG. 3 shows that the organic additive methyl benzoate has the best optimization effect. The refractive index modulation of the prepared volume holographic grating can reach 0.08, which is much higher than that of the comparative example 1 without organic additive. This may be because methyl benzoate has a strongly polar ester group at one end and a weakly polar aryl group at the other end. The other two organic additives, methyl 1-cyanopropanoate and α-methylstyrene, also have a certain optimizing effect on the refractive index modulation of volume holographic gratings. In general, the introduced organic additives have a good optimizing effect on volume holographic gratings, have practical effects, and are beneficial for the preparation of high-optical-performance AR/VR display devices.

As described above, the present disclosure provides a raw material system for a volume holographic grating, a volume holographic grating, and a display element. Based on the raw material system composed of the photopolymerizable monomer, the liquid crystal and the photoinitiator, and an organic additive is introduced in the present disclosure. The organic additive can promote phase separation of a PDLC (Polymer Dispersed Liquid Crystal) film during exposure, increase the refractive index difference between the bright region and the dark region, thus improving the refractive index modulation of the volume holographic grating to meet the requirements of display applications in AR/VR fields. The organic additive introduced in the present disclosure has the characteristics of simple structure, expandable structure, and good light/thermal stability. Adding the organic additive into PDLC components can significantly improve the Δn value. Compared with conventional methods, the embodiments of the present disclosure do not require complex molecular design or lengthy multi-component optimization, have better universality, and can further improve the optical performance of the volume holographic grating.

It should be understood that the application of the present disclosure is not limited to the above embodiments. Those skilled in the art can make modifications or variations based on the above description, and all such modifications and variations shall fall within the protection scope of the appended claims of the present disclosure.

## Claims

1. A raw material system for a volume holographic grating, comprising a photopolymerizable monomer, a liquid crystal, an organic additive and a photoinitiator; wherein the organic additive is a compound of Formula A-B, wherein A is a group having affinity for photopolymerizable monomers, and B is a group having affinity for liquid crystals.

2. The raw material system for the volume holographic grating according to claim 1, wherein the group having affinity for photopolymerizable monomers is an ester group.

3. The raw material system for the volume holographic grating according to claim 1, wherein the group having affinity for liquid crystals is selected from the group consisting of phenyl group, fluoro-substituted phenyl group, cyano-substituted phenyl group, trifluoromethyl-substituted phenyl group, methyl-substituted phenyl group, thienyl group, pyrrolyl group, cyano-substituted alkyl group and halogen-substituted alkyl group.

4. The raw material system for the volume holographic grating according to claim 2 or 3, wherein the organic additive is one or more compounds selected from the group consisting of Formulas:

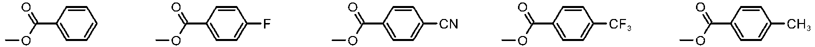

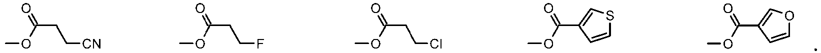

5. The raw material system for the volume holographic grating according to claim 1, wherein the group having affinity for photopolymerizable monomers is selected from the group consisting of alkene-containing group, carbonyl-containing group and alkyl ether group.

6. The raw material system for the volume holographic grating according to claim 5, wherein the alkene-containing group is an allyl group.

7. The raw material system for the volume holographic grating according to claim 5, wherein the carbonyl-containing group is selected from the group consisting of ethyl ketone group, acetaldehyde group and acetamido group.

8. The raw material system for the volume holographic grating according to claim 1, wherein the group having affinity for liquid crystals is a phenyl group.

9. The raw material system for the volume holographic grating according to any one of claims 5 to 8, wherein the organic additive is one or more compounds selected from the group consisting of Formulas:

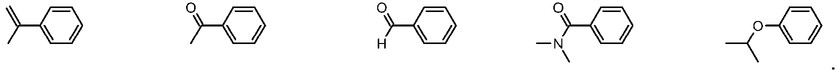

10. The raw material system for the volume holographic grating according to claim 1, wherein based on the total weight of the raw material system for the volume holographic grating, the weight percentage of the photopolymerizable monomer is 30%-50%, the weight percentage of the liquid crystal is 30%-50%, the weight percentage of the organic additive is 1%-10%, and the weight percentage of the photoinitiator is 1%.

11. The raw material system for the volume holographic grating according to claim 1, wherein the photopolymerizable monomer is at least one selected from the group consisting of acrylates and acrylate derivatives.

12. The raw material system for the volume holographic grating according to claim 11, wherein the acrylate derivative is at least one selected from the group consisting of 2-hydroxy-3-acrylic acid propyl ester, methyl acrylate, methyl methacrylate, deuterated methyl methacrylate, fluoro-substituted methyl methacrylate, ethyl acrylate, ethyl methacrylate, ethyl ethyl acrylate and propyl ethyl acrylate.

13. The raw material system for the volume holographic grating according to claim 1, wherein the liquid crystal is a nematic liquid crystal.

14. The raw material system for the volume holographic grating according to claim 13, wherein the nematic liquid crystal is at least one selected from the group consisting of 4-cyano-4'-pentylbiphenyl, 5-octylphenyl-2-methoxyphenyl-4-pyridone and liquid crystal mixture E7.

15. The raw material system for the volume holographic grating according to claim 1, wherein the photoinitiator is a mixture of dibromofluorescein and phenylglycine, or a mixture of Acid Red 94 and phenylglycine.

16. A volume holographic grating prepared by subjecting the raw material system for the volume holographic grating according to any one of claims 1 to 15 to coherent light exposure.

17. A preparation method for a volume holographic grating, comprising: subjecting the raw material system for the volume holographic grating according to any one of claims 1 to 15 to coherent light exposure to obtain the volume holographic grating.

18. The preparation method of a volume holographic grating according to claim 17, wherein the method comprises the following steps:
placing the raw material system for the volume holographic grating in a round-bottom flask and stirring uniformly;
filling the uniformly mixed raw material system for the volume holographic grating into a liquid crystal cell by capillary action;
edge-sealing the liquid crystal cell to obtain a standard holographic exposure device; and
performing exposure with a standard holographic exposure device to prepare the volume holographic grating.

19. The preparation method for the volume holographic grating according to claim 18, wherein the liquid crystal cell includes at least one glass substrate.

20. The preparation method for the volume holographic grating according to claim 18, wherein the exposure is red light exposure.
